# FASCICULE DE BREVET EUROPEEN

(11) **EP 1 286 660 B1**
(45) Date de publication et mention de la délivrance du brevet: **27.09.2006**
(21) Numéro de dépôt: 01943573.4
(22) Date de dépôt: 06.06.2001
(51) Int. Cl.: A61K 9/107

(54) **COMPOSITION PHARMACEUTIQUE SOUS FORME LIQUIDE DESTINEE A L'ADMINISTRATION PAR VOIE ORALE D'UN PRINCIPE ACTIF, AYANT UN GOUT DESAGREABLE, NOTAMMENT UN GOUT AMER**
FLÜSSIGE ARZNEIZUBEREITUNGEN FÜR DIE ORALE VERABREICHUNG VON UNANGENEHM, BITTERSCHMECKENDEN ARZNEIMITTELN
PHARMACEUTICAL COMPOSITION IN LIQUID FORM FOR ORAL ADMINISTRATION OF AN ACTIVE PRINCIPLE, WITH UNPLEASANT, IN PARTICULAR BITTER, TASTE

(30) Priorité: 08.06.2000 FR 0007364
(43) Date de publication de la demande: 05.03.2003
(73) Titulaire: LABORATOIRES CRINEX, 92120 Montrouge (FR)
(72) Inventeur: VANDAMME, Thierry, F-67200 Strasbourg (FR)
(74) Mandataire: Bernasconi, Jean Raymond
(86) Numéro de dépôt international: PCT/FR2001/001748
(87) Numéro de publication internationale: WO 2001/093834

(56) Documents cités:
- WO-A-01/01960
- GB-A- 2 331 458

## Description

La présente invention concerne une composition pharmaceutique sous forme liquide destinée à l'administration par voie orale d'un principe actif, ayant un goût désagréable, notamment un goût amer.

De nombreux médicaments ont un goût amer désagréable en particulier lorsqu'ils sont administrés sous une forme liquide, qui est une forme appropriée notamment à être administrée à des enfants ou des personnes âgées ayant des problèmes de déglutition de formes solides tels que comprimés, gélules, etc.

Pour cette raison, les formes pharmaceutiques liquides et fluides tels que sirops, potions, émulsions, suspensions sont préférées. Néanmoins, l'administration de principes actifs ayant un goût désagréable à l'aide de ces formes pharmaceutiques s'avère difficile et conduit à une diminution de la compliance de telles formulations en particulier chez des catégories de patients mentionnés ci-dessus. Afin de masquer le goût désagréable de principes actifs hydrosolubles tels que du chlorhydrate de ranitidine, différentes approches ont été développées.

La demande de brevet WO 99/53 902 décrit des compositions pharmaceutiques administrables par voie orale comprenant des polysaccharides réticulés. Ces compositions ont la propriété de masquer le goût de principes actifs désagréables et d'assurer une libération des principes actifs. Les polysaccharides réticulés sont réalisés à partir d'acide alginique, de pectine déméthylées avec en outre un polymère qui peut être digéré tel que de l'amidon, des dérivés d'amidon, des α-glycanes, des peptides et des polypeptides.

La demande de brevet WO 89/05 640 décrit des granules pharmaceutiques renfermant de la cimétidine et comme agent de granulation et de masquage de goût un ester d'un composé polyhydroxylé ayant un goût agréable et accepté dans le domaine de la pharmacie. Des esters du glycérol sont cités et décrits comme adjuvant dans des préparations de type comprimés à mâcher qui ont un goût agréable et une bonne biodisponibilité.

La demande de brevet WO 99/52 556 décrit des techniques permettant de masquer le goût amer d'ions métalliques ou d'autres composés tels que caféine, menthol. La technique consiste à réaliser un mélange d'acides aminés, d'acides organiques et/ou inorganiques avec ou sans les ions métalliques. Cette technique peut également être mise à profit pour masquer le goût désagréable de principes actifs pharmaceutiques, de vitamines, d'enzymes purifiées, de cellules, de virus, d'anticorps, de protéines, de fongicides et pesticides.

Le brevet EP 0 717 992 décrit une composition pharmaceutique sous forme de suspension aqueuse ainsi que des méthodes de préparation. La suspension comprend une quantité de principe actif en suspension dans un véhicule aqueux contenant des additifs stabilisants, de l'eau et des adjuvants de masquage de goût.

La demande de brevet WO 93/24 109 décrit des compositions pharmaceutiques ayant un goût agréable et constituées de granulés enrobés à l'aide de copolymères d'esters méthacryliques comme agent de masquage de goût. Les granulés peuvent être utilisés pour la préparation de comprimés à mâcher qui présentent un goût agréable et une bonne biodisponibilité. La libération du ou des principes actifs des comprimés est assurée par diffusion à travers l'enrobage et est influencée par la solubilité du principe actif et du milieu ambiant. Les copolymères préférés sont les poly(éthacrylate) et poly(méthacrylate).

La demande de brevet EP 0 296 119 décrit des compositions de mousses aérosols huileuses et une méthode de préparation de celles-ci. Cette mousse est réalisée en incorporant un agent complexant inorganique, de l'eau, un agent édulcorant, un agent de masquage de goût et un gaz propulseur. La préparation peut renfermer jusqu'à 80 % d'huile.

La présente invention a pour but de fournir une composition destinée à masquer le goût désagréable de médicaments administrés par voie orale, et plus généralement de substances hydrosolubles prévues pour être ingérées par voie buccale.

L'invention repose sur la découverte que le goût désagréable de substances actives, hydrosolubles, notamment de médicaments, peut être masqué en incorporant les substances actives dans la phase aqueuse d'une microémulsion eau-dans-huile.

Sans vouloir être liés par une théorie précise, l'auteur estime que la présence d'une phase externe lipidique permet le recouvrement des papilles et des récepteurs présents sur la langue et empêche ainsi le contact direct de la substance active dissoute dans l'eau avec ces papilles et récepteurs.

L'invention a donc pour objet l'utilisation d'une microémulsion stable de type eau-dans-huile pour masquer le goût désagréable de substances actives hydrosolubles, notamment de médicaments, destinées à être administrés par voie orale.

EP 334 777 décrit des microémulsions à usage cosmétique ou pharmaceutique comprenant un agent tensioactif qui est un ester mixte de glycérol et de polyéthylèneglycol, un agent cotensioactif qui est un ester de polyglycérol et d'un acide gras, le constituant de la phase lipidique ayant une balance hydrophile-lipopile (HBL) d'au plus trois.

Ces microémulsions sont décrites comme ayant une stabilité élevée et une bonne tolérance cutanée.

FR 2 710 535 décrit une composition à usage pharmaceutique ou cosmétique destinée à former *in situ* une microémulsion avec le liquide biologique de l'organisme, à la température du corps humain et destinée à augmenter la biodisponibilité du ou des principes actifs.

La composition comprend une phase lipophile constituée par des composés polyglycolysés saturés en C₈-C₁₈, présentant une balance hydrophile-lipophile (HLB) comprise entre 6 et 16, représentant de 30 à 75 % du poids total de la composition, un agent tensioactif (TA) choisi dans le groupe comprenant les glycérides polyglycolysés saturés en C₈-C₁₈ et les esters oléiques du polyglycérol, présentant une HLB comprise entre 6 et 16, un agent co-tensioactif (CoTA) choisi dans le groupe comprenant les esters lauriques du propylène glycol, les esters oléiques du polyglycérol et l'éthyl diglycol ; et dans laquelle le rapport TA/CoTA est compris entre 0,5 et 4.

Une microémulsion est une émulsion homogène, fluide et stable, composée de quatre constituants essentiels, respectivement une phase aqueuse, une phase lipidique, au moins un agent tensioactif (TA) et au moins un agent cotensioactif (CoTA), dans laquelle les gouttelettes formant la phase interne sont de taille micrométrique, généralement non supérieure à 100 nm.

Les microémulsions ont été largement étudiées. Il n'y a donc pas lieu de les décrire ici en détail. Pour les principes de base, on pourra se reporter à l'ouvrage de Bourrel M. D. Schechter R.S. intitulé "Microemulsions and related systems".

Un agent tensioactif est un composé chimique possédant deux groupements, le premier polaire ou ionique, qui présente une grande affinité pour l'eau, le second hydrophobe comprenant notamment une chaîne aliphatique plus ou moins longue, ce composé ayant la propriété de former des micelles.

Un agent cotensioactif est également un composé chimique partiellement soluble dans la phase eau et dans la phase huile à caractère plus hydrophobe que l'agent tensioactif, destiné à provoquer la solubilisation mutuelle des phases aqueuses et huileuses dans une microémulsion, la synergie résultant de l'action complémentaire du tensioactif initial et du cotensioactif étant indispensable à l'obtention des microémulsions.

Des cotensioactifs sont connus et décrits notamment dans l'ouvrage précité de Bourrel et Schechter.

Pour les constituants de la phase lipidique, on utilise un constituant ou un mélange de constituants à goût neutre, hydrodispersible dans l'eau et compatibles avec une administration orale.

Avantageusement, le constituant ou le mélange de constituants formant la phase lipidique est liquide à température ambiante, c'est-à-dire à des températures comprises généralement entre 15 et 40°C.

Des constituants particulièrement avantageux pour la phase lipidique sont les esters d'acide gras de polyols, éventuellement polyéthoxylés.

Les acides gras peuvent être saturés ou insaturés, linéaires ou ramifiés et comprennent de préférence de 12 à 18 atomes de carbone.

On peut citer en particulier les acides oléique, laurique, palmitique et stéarique.

Le polyol peut être notamment le glycérol ou un dérivé de polycondensation de celui-ci, le degré de polycondensation variant en général de 2 pour le diglycérol à 10 pour le triacontaglycérol.

Le polyol peut également de façon avantageuse être le sorbitane.

Les esters polyéthoxylés de sorbitane comprennent généralement de 5 à 40, de préférence environ 20 groupes d'oxyde d'éthylène par molécule de polyol.

Les esters peuvent être des mono- ou polyesters des polyols précédemment décrits.

Une microémulsion particulièrement homogène et stable est obtenue lorsque les constituants formant la phase lipidique ont une balance hydrophile-lipophile (HLB) comprise entre 1,8 et 8,6, de préférence entre 4 et 8,6.

Comme constituant de la phase lipidique, on peut citer les esters de glycérol éthoxylés commercialisés sous la dénomination LABRAFIL® par la société Gattefossé, France, en particulier le LABRAFIL® M 1944 CS qui est un ester oléique de glycérol polyéthoxylé, comportant en moyenne six groupes oxyde d'éthylène par molécule de glycérol.

Conviennent également bien les esters de sorbitane sous forme liquide, en particulier ceux commercialisés sous la dénomination SPAN®. On peut citer notamment les SPAN® 20 (monolaurate de sorbitane, HLB = 8,6), SPAN® 80 (mono-oléate de sorbitane, HLB = 4,3), et SPAN® 85 (trioléate de sorbitane, HLB = 1,8).

La phase lipidique constitue avantageusement de 10 à 72 % du volume total de la composition.

Elle peut renfermer outre des constituants lipophiles à proprement parler, des agents antioxydants tels que du gallate de propyle, et des agents conservateurs tels que les esters de l'acide parahydroxybenzoïque (parahydroxybenzoate de méthyle, Nipagine®, parahydroxybenzoate de propyle, Nipasol®), de manière à éviter la prolifération de microorganismes, de moisissures, ou de champignons ou des agents viscosifiants tels que de la silice colloïdale (Cab-O-Sil®, Aérosil®) destinés à augmenter la viscosité de la phase lipophile.

La phase aqueuse renfermant le ou les principes actifs peut renfermer d'autres substances hydrosolubles telles que des tampons pour stabiliser le ou les principes actifs dissous dans la solution au pH optimum de stabilité, des édulcorants tels que le saccharose, le cyclamate de sodium, la saccharine, le saccharinate de sodium, le glycyrhizinate de sodium, etc... pour améliorer le goût de la préparation, des arômes naturels ou artificiels hydrophiles tels que des arômes de fraise, framboise, etc... pour améliorer le goût de la préparation, des agents conservateurs tels que parahydroxybenzoate de méthyle ou de propyle, de l'acide sorbique, des agents antioxydants tels que le métabisulfite de sodium, des agents solubilisants tels que de l'éthanol, le glycérol, les cyclodextrines, etc... pour améliorer la solubilité du ou des principes actifs dans la phase aqueuse.

La phase aqueuse peut renfermer également des agents viscosifiants solubles dans l'eau tels que de 1 % à 30 % en poids, de dérivés d'origine naturelle (gomme xanthane, gomme arabique, etc), hémisynthétique tels que des dérivés cellulosiques (hydroxypropylcellulose, méthylcellulose, ...) ou d'autres polymères d'origine synthétique (alcool polyvinylique, polyéthylène glycol et copolymères, etc...) destinés à augmenter la viscosité de la phase aqueuse ou à faciliter la solubilisation du ou des principes actifs.

La phase aqueuse constitue avantageusement de 1 à 30 %, de préférence de 1 à 10 % du volume total de la composition.

L'agent tensioactif est avantageusement un composé non ionique, liquide à température ambiante, ayant une balance hydrophile-lipophile (HLB) comprise entre 10,5 et 15,6, un goût neutre et compatible avec un usage oral.

Les meilleurs résultats sont obtenus lorsque l'agent tensioactif est choisi parmi les esters d'acide gras de polyols éventuellement polyéthoxylés.

Un premier groupe d'agents tensioactifs préféré est constitué par les esters d'acide gras en C₆-C₁₈, insaturés ou saturés, linéaires ou ramifiés, du glycérol.

Il peut s'agir de mono-, di- ou tri-esters d'acide gras identiques ou différents, ou de mélanges de ceux-ci.

Un second groupe d'agents tensioactifs préféré est constitué par les esters de sorbitane polyéthoxylés ayant un degré d'éthoxylation moyen compris entre 5 et 40, de préférence environ 20 molécules d'oxyde d'éthylène par molécule de sorbitane.

Il peut là également s'agir de mono-, di-, ou tri-esters de sorbitane polyéthoxylé avec un ou plusieurs acides gras en C₁₂-C₁₈ identiques ou différents tels que définis ci-dessus. Les monoesters ou mélange de monoesters de sorbitane et d'acide gras sont toutefois préférés.

Un groupe particulier d'agents tensioactifs particulièrement appropriés au but de l'invention est constitué par la série des TWEEN®.

On peut citer notamment les tensioactifs suivants : TWEEN® 40, TWEEN® 60, TWEEN® 80 et TWEEN® 85, ayant respectivement des valeurs HBL de 15,6 ; 14,9 ; 15,0 et 11,0.

L'agent tensioactif est généralement présent à raison d'au moins 18 %, avantageusement au moins 25 % en poids de la composition et jusqu'à 78 %, avantageusement jusqu'à 53 %, en poids de la composition.

L'agent co-tensioactif (CoTA) est avantageusement un alcool, comportant un ou plusieurs groupes hydroxylés compatible avec un usage oral. Il peut s'agir d'un monoalcool, par exemple le butanol, d'un diol par exemple le propylène glycol, d'un polyol, par exemple le glycérol ou d'un éther de polyol notamment le glycérol monoéthyléther.

Un rapport volumique TA/CoTA préféré est compris entre 1/1 et 50/1, avantageusement 1/1 et 10/1.

L'ensemble agent tensioactif-agent-co-tensioactif représente de préférence de 30 à 80 % du volume total de la composition.

Le principe actif est un principe actif hydrosoluble.

Par "hydrosoluble", on entend que le composé est soluble dans l'eau à 20°C en une quantité d'au moins une partie de principe actif dans 100 parties de phase aqueuse.

Des exemples de tels principes actifs sont des antagonistes du récepteur H₂, des anti-inflammatoires, des vasodilatateurs coronariens, des vasodilatateurs cérébraux, des vasodilatateurs périphériques, des psychotropes, des composés à activité stimulante, des anti-histaminiques, des laxatifs, des décongestionnants, des vitamines, des sédatifs gastro-intestinaux, des préparations anti-diarrhées, des médicaments anti-angoreux, des anti-arythmiques, des anti-hypertenseurs, des vaso-constricteurs, des anti-migraineux, des anti-coagulants, des anti-thrombotiques, des analgésiques, des anti-pyrétiques, des hypnotiques, des sédatifs, des anti-émétiques, des anti-nausées, des anti-convulsivants, des substances à activité neuromusculaire, des agents hyper- ou hypoglycémiants, des préparations à activité thyroïdienne ou anti-thyroïdienne, des diurétiques, des antispasmodiques, des relaxants de l'utérus, des additifs minéraux, des additifs alimentaires, des médicaments contre l'obésité, des anaboliques, des substances à activité de type érythropoïétine, des anti-asthmatiques, des bronchodilatateurs, des expectorants, des suppresseurs de toux, des mucolytiques et des médicaments anti-uricémiants.

De préférence, la substance active est une substance pharmaceutiquement active comprenant un antagoniste des récepteurs H₂. Ceux-ci sont de préférence choisis parmi les dérivés hydrosolubles de la cimétidine, de la ranitidine, de la nizatidine et de la famotidine.

On peut citer en particulier les chlorhydrates de ces composés en particulier le chlorhydrate de ranitidine ou N-[2-[[[5-[(diméthylamino)méthyl]-2-furanyl]méthyl]thio]éthyl]-N'-méthyl-2-nitro-1,1-éthane diamine.

Des exemples de médicaments également appropriés au but de l'invention sont les dérivés solubles des principes actifs suivants : piroxicam, chlorophéniramine, clarithromycine, ibuprofène, ketoprofène, diclofénac, ambroxol, diphénhydramine, diltiazem, alprazolam, phénylpropanolamine, guaïphénésine, bromophéniramine, métoclopramide, tropisétron, andansétrol, caféine, oxybutynine, ticlopidine, roxythromycine, éthromycine, azithromycine.

La substance active peut également être un agent de rafraîchissement d'haleine ou un agent anti-microbien, plus généralement un agent destiné à l'hygiène buccale, ou encore un agent diététique ou nutritionnel.

Il peut s'agir en particulier de sels de cations métalliques polyvalents utilisés pour rafraîchir l'haleine, par exemple le salicylate et le gluconate de zinc, des sels d'argent tels que le nitrate et le gluconate d'argent. Lorsqu'il s'agit d'agents anti-microbiens, ceux-ci sont en particulier l'acide benzoïque et ses dérivés qui ont un goût désagréable, en particulier lorsqu'ils sont utilisés en concentration élevée.

Les agents anti-microbiens incluent également les composés d'ammonium quaternaire, tels que les halogénures d'alkyl pyridinium, par exemple le chlorure de cétylpyridinium, le chlorure de benzéthonium et le chlorure de benzalkonium, les bis-biguanines tels que le chlorhexydène [1,1'-hexaméthylènebis(5-(4-chlorophényl)biguinanine].

Les compositions selon l'invention peuvent comprendre jusqu'à 30 g de principe actif par 100 ml de solution microémulsionnée.

Des quantités préférées sont situées entre 1 mg et 10 g par 100 ml de solution microémulsionnée.

Les compositions selon l'invention peuvent être obtenues par un procédé comprenant les étapes consistant à :
- dissoudre le principe actif dans la phase aqueuse, de préférence tamponnée contenant le cas échéant les ingrédients et additifs mentionnés ci-dessus ;
- ajouter à la phase aqueuse contenant le principe actif, le cotensioactif et homogénéiser l'ensemble ;
- dissoudre dans la solution précédemment obtenue le tensioactif;
- ajouter à cette préparation les constituants de la phase lipidique et émulsionner puis homogénéiser l'ensemble.

Pour la préparation de la composition, on utilise des constituants ayant une quasi-absence de toxicité, c'est-à-dire présentant une DL₅₀ très élevée, généralement supérieure à plusieurs grammes par kg de poids corporel.

Pour que les constituants aient une bonne innocuité, ils doivent être préparés avec des matières premières aussi pures que possible et être purifiés par la suite.

Le mélange de ces différents constituants s'effectue dans des proportions préalablement déterminées par l'expérience, sans précaution particulière ou ordre spécifique, notamment à température ambiante, sous faible agitation.

L'invention a également pour objet un procédé de masquage du goût d'un principe actif hydrosoluble à goût désagréable destiné à une administration orale, caractérisé en ce que ledit principe actif est dissous avant administration dans la phase aqueuse d'une microémulsion de type eau-dans-huile.

Certaines compositions de l'invention sont nouvelles en tant que telles et constituent un autre objet de l'invention.

L'invention a ainsi également pour objet une composition pour l'administration par voie orale de substances actives hydrosolubles ayant un goût désagréable consistant en une microémulsion de type eau-dans-huile comprenant :
- une phase aqueuse contenant un principe actif hydrosoluble représentant de 1 à 30 % en volume total de la composition ;
- une phase lipidique constituée d'au moins un ester d'un acide gras en C₁₂-C₁₈ et d'un polyol, ayant une balance hydrophile-lipophile (HLB) comprise entre 1,8 et 8,6, la phase lipidique représentant de 10 à 60 % du volume total de la composition ;
- au moins un agent tensioactif (TA) ayant une balance hydrophile-lipophile (HLB) comprise entre 10,5 et 15,6, choisi parmi les esters d'acide gras et de polyol, éventuellement éthoxylés ;
- au moins un agent co-tensioactif (CoTA) consistant en un alcool; le rapport volumique TA/CoTA étant compris entre 1 et 50 ; sous réserve que la phase hydrophile ne soit pas apportée par un organisme vivant auquel la composition est administrée.

L'invention a en outre pour objet une composition pharmaceutique comprenant une microémulsion de type eau-dans-huile et un sel hydrosoluble de ranitidine, notamment le chlorhydrate de ranitidine dissous dans la phase aqueuse de ladite microémulsion.

La composition de l'invention comprend le sel de ranitidine en une quantité comprise entre 1 mg/ml et 30 mg/ml, de préférence entre 5 mg/ml et 10 mg/ml.

On décrira ci-après en détails certains modes de réalisation particuliers de l'invention.

On se référera pour l'exemple 15 aux figures annexées sur lesquels :
- la figure 1 représente la zone d'existence de microémulsion dans un diagramme ternaire selon l'invention dans laquelle le rapport TA/CoTA est de 1,0 ;
- la figure 2 représente la zone d'existence de microémulsion dans un diagramme ternaire selon l'invention dans laquelle le rapport TA/CoTA est de 50,0.

### EXEMPLE 1 :

1000 mg de chlorhydrate de ranitidine sont dissous dans 10 ml de tampon phosphate pH 6,4. 13,3 ml de diéthylène glycol monoéthyléther (Transcutol® P) sont ajoutés et l'ensemble est homogénéisé. 26,6 ml de sorbitane mono-oléate polyoxyéthylène (Tween® 80) sont dissous dans la solution obtenue précédemment. 50 ml d'oléyl macrogol-6 glycérides (Labrafil® M 1944 CS) sont ajoutés à la préparation et sont émulsionnés. Le tout est homogénéisé. Une préparation jaune pâle est obtenue. Le rapport TA/CoTA est de 2.

### EXEMPLE 2 :

1000 mg de chlorhydrate de ranitidine sont dissous dans 10 ml de tampon phosphate pH 7,2 renfermant 2 mg de parahydroxy-benzoate de propyle, 10 mg de parahydroxybenzoate de méthyle et 400 mg de cyclamate de sodium. 16,67 ml de propylène glycol sont dissous et l'ensemble est homogénéisé. 37,05 ml de sorbitane mono-oléate polyoxyéthylène (Tween® 80) sont dissous dans la solution obtenue précédemment. 32,27 ml d'oléyl macrogol-6 glycérides (Labrafil® M 1944 CS) renfermant 46 mg de gallate de propyle et 4,0 ml d'huile essentielle de citron sont ajoutés à la préparation et sont émulsionnés. Le tout est homogénéisé. Une préparation jaune pâle est obtenue. Le rapport TA/CoTA est de 2,22.

### EXEMPLE 3 :

1000 mg de chlorhydrate de ranitidine sont dissous dans 10 ml de solution aqueuse contenant 0,288 g de citrate trisodique, 2 mg de parahydroxy benzoate de propyle, 10 mg de parahydroxybenzoate de méthyle et 400 mg de cyclamate de sodium. 16,67 ml de propylène glycol sont dissous dans la solution précédente et l'ensemble est homogénéisé. 37,05 ml de sorbitane mono-oléate polyoxyéthylène (Tween® -80) sont dissous dans la solution obtenue précédemment. 32,27 ml d'oléyl macrogol-6 glycérides (Labrafil® M 1944 CS) renfermant 46 mg de gallate de propyle et 4,0 ml d'huile essentielle de citron sont ajoutés à la préparation et sont émulsionnés. Le tout est homogénéisé. Une préparation jaune pâle est obtenue. Le rapport TA/CoTA est de 2,22.

### EXEMPLE 4 :

1000 mg de chlorhydrate de ranitidine sont dissous dans 10 ml de tampon phosphate pH 6,4. 10 ml de diéthylène glycol monoéthyléther (Transcutol® P) sont dissous et l'ensemble est homogénéisé. 40 ml de sorbitane mono-oléate polyoxyéthylène (Tween® 80) sont dissous dans la solution obtenue précédemment. 40 ml d'oléyl macrogol-6 glycérides (Labrafil® M 1944 CS) sont ajoutés à la préparation et sont émulsionnés. Le tout est homogénéisé. Une préparation jaune pâle est obtenue. Le rapport TA/CoTA est de 4.

### EXEMPLE 5 :

1000 mg de chlorhydrate de ranitidine sont dissous dans 10 ml de tampon phosphate pH 6,4. 3,3 ml de diéthylène glycol monoéthyléther (Transcutol® P) sont dissous et l'ensemble est homogénéisé. 56,7 ml de sorbitane mono-oléate polyoxyéthylène (Tween® 80) sont dissous dans la solution obtenue précédemment. 30 ml d'oléyl macrogol-6 glycérides (Labrafil® M 1944 CS) sont ajoutés à la préparation et sont émulsionnés. Le tout est homogénéisé. Une préparation jaune pâle est obtenue. Le rapport TA/CoTA est de 17,18.

### EXEMPLE 6 :

1000 mg de chlorhydrate de ranitidine sont dissous dans 10 ml de tampon phosphate pH 6,4. 10 ml de diéthylène glycol monoéthyléther (Transcutol® P) sont dissous et l'ensemble est homogénéisé. 30 ml de sorbitane mono-oléate polyoxyéthylène (Tween® 80) sont dissous dans la solution obtenue précédemment. 0,5 g d'Aérosil® 200 dissous dans 50 ml d'oléyl macrogol-6 glycérides (Labrafil® M 1944 CS) sont ajoutés à la préparation et sont émulsionnés. Le tout est homogénéisé. Une préparation jaune pâle est obtenue. Le rapport TA/CoTA est de 3.

### EXEMPLE 7 :

1000 mg de chlorhydrate de ranitidine sont dissous dans 10 ml de ampon phosphate pH 6,4 renfermant 400 mg de cyclamate de sodium, 2 mg de parahydroxybenzoate de propyle et 10 mg de parahydroxybenzoate de méthyle. 16,67 ml de propylène glycol sont dissous et l'ensemble est homogénéisé. 37,05 ml de sorbitane mono-oléate polyoxyéthylène (Tween® 80) sont dissous dans la solution obtenue précédemment. 46 mg de gallate de propyle sont dissous dans 32,27 ml de polyglycéryl-6-dioléate (Plurol® oléique CC497) chauffés à 50°C, sont ajoutés à la préparation et sont émulsionnés. 4 ml d'huile esentielle de citron sont ajoutés et le tout est homogénéisé. Une préparation jaune pâle est obtenue. Le rapport TA/CoTA est de 2,22.

### EXEMPLE 8 :

1000 mg de chlorhydrate de ranitidine sont dissous dans 10 ml de tampon phosphate pH 6,4 renfermant 400 mg de cyclamate de sodium, 2 mg de parahydroxybenzoate de propyle et 10 mg de parahydroxybenzoate de méthyle. 16,67 ml de propylène glycol sont dissous et l'ensemble est homogénéisé. 37,05 ml de sorbitane mono-oléate polyoxyéthylène (Tween® 80) sont dissous dans la solution obtenue précédemment. 46 mg de gallate de propyle sont dissous dans 32,27 ml de Span® 20 chauffés à 50°C, sont ajoutés à la préparation et sont émulsionnés. 4 ml d'huile esentielle de citron sont ajoutés et le tout est homogénéisé. Une préparation jaune pâle est obtenue. Le rapport TA/CoTA est de 2,22.

### EXEMPLE 9 :

800 mg de chlorhydrate de ranitidine sont dissous dans 10 ml de tampon phosphate pH 6,4. 17 ml de diéthylène glycol monoéthyléther (Transcutol® P) sont dissous et l'ensemble est homogénéisé. 31 ml de caprylocaproyl Macrogol-8 glycérides (Labrasol®) sont dissous dans la solution obtenue précédemment. 21 ml d'oléoyl macrogol-6 glycérides (Labrafil® M 1944 CS) et 21 ml de polyglycéryl-6-dioléate (Plurol® oléique CC 497) sont ajoutés à la préparation et sont émulsionnés. Le tout est homogénéisé. Une préparation jaune pâle est obtenue. Le rapport TA/CoTA est de 1,82.

### EXEMPLE 10 :

800 mg de chlorhydrate de ranitidine sont dissous dans 10 ml de tampon phosphate pH 6,4. 17 ml de diéthylène glycol monoéthyléther (Transcutol® P) sont dissous et l'ensemble est homogénéisé. 31 ml de caprylocaproyl Macrogol-8 glycérides (Labrasol®) sont dissous dans la solution obtenue précédemment. 42 ml de polyglycéryl-6-dioléate (Plurol® oléique CC 497) sont ajoutés à la préparation et sont émulsionnés. Le tout est homogénéisé. Une préparation jaune pâle est obtenue. Le rapport TA/CoTA est de 1,82.

### EXEMPLE 11 :

1000 mg de chlorhydrate de ranitidine sont dissous dans 10 ml de tampon phosphate pH 7,0 renfermant 10 mg de parahydroxybenzoate de méthyle et 2 mg de parahydroxybenzoate de propyle. 400 mg de cyclamate de sodium sont dissous dans la phase aqueuse. 13,3 ml de diéthylène glycol monoéthyléther (Transcutol® P) sont dissous et l'ensemble est homogénéisé. 26,6 ml de sorbitane mono-oléate polyoxyéthylène (Tween® 80) sont dissous dans la solution obtenue précédemment. 5 ml d'huile essentielle de citron et 45 mg de gallate de propyle sont dissous dans 45 ml d'oléoyl macrogol-6glycérides (Labrafil® M 1944 CS) ; l'ensemble est ajouté à la préparation obtenue précédemment et est émulsionné. Le tout est homogénéisé. Une préparation jaune pâle est obtenue. Le rapport TA/CoTA est de 2.

### EXEMPLE 12 :

800 mg de chlorhydrate de ranitidine sont dissous dans 5 ml de tampon phosphate pH 6,4. 1 ml de glycérine est dissous et l'ensemble est homogénéisé. 50 ml de sorbitane mono-oléate polyoxyéthylène (Tween® 80) sont dissous dans la solution obtenue précédemment. 44 ml de Labrafil® M 1944 CS sont ajoutés au mélange précédent et le tout est émulsionné et homogénéisé. Une préparation jaune pâle est obtenue. Le rapport TA/CoTA est de 50.

### EXEMPLE 13 :

800 mg de chlorhydrate de ranitidine sont dissous dans 10 ml de tampon phosphate pH 6,4. 10 ml de polyéthylène glycol sont dissous et l'ensemble est homogénéisé. 40 ml de sorbitane mono-oléate polyoxyéthylène (Tween® 80) sont dissous dans la solution obtenue précédemment. 40 ml de Labrafil® M 1944 CS sont ajoutés au mélange précédent et le tout est émulsionné et homogénéisé. Une préparation jaune pâle est obtenue. Le rapport TA/CoTA est de 4.

### EXEMPLE 14 :

800 mg de chlorhydrate de ranitidine sont dissous dans 10 ml de tampon phosphate pH 6,4. 10 ml de butanol tertiaire sont dissous et l'ensemble est homogénéisé. 40 ml de sorbitane mono-oléate polyoxyéthylène (Tween® 80) sont dissous dans la solution obtenue précédemment. 40 ml de Labrafil® M 1944 CS sont ajoutés au mélange précédent et le tout est émulsionné et homogénéisé. Une préparation jaune pâle est obtenue. Le rapport TA/CoTA est de 4.

### EXEMPLE 15 :

On a déterminé les zones d'existence de microémulsions dans lesquelles la phase lipidique était constituée de Labrafil® M 1944 CS, l'agent tensioactif de Tween® 80 et l'agent co-tensioactif de Transcutol® P.

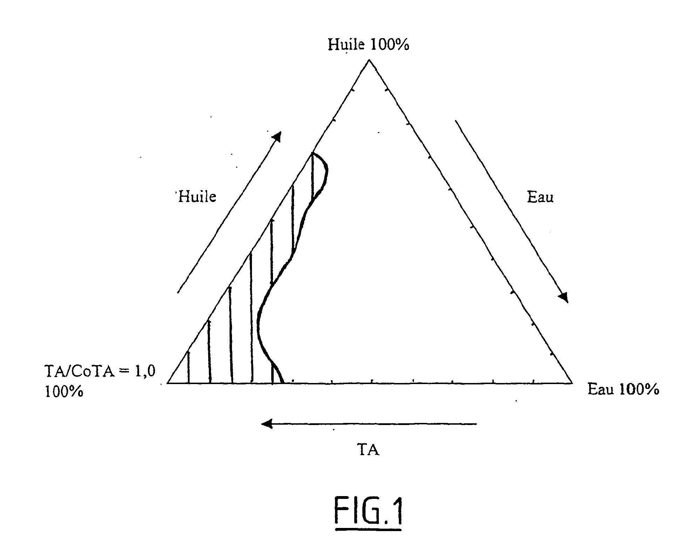

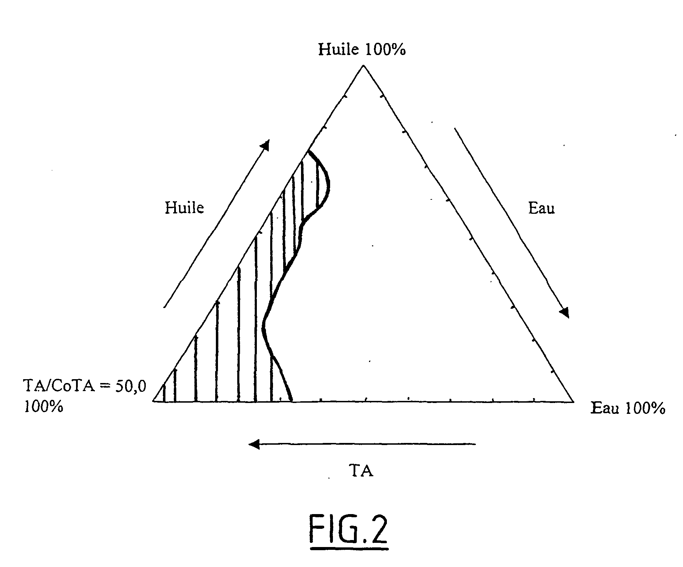

Pour cela, on a tracé des diagrammes pseudo-ternaires, en conservant un rapport TA/CoTA de 1 (figure 1) et de 50 (figure 2). On a réalisé des mélanges phase lipidique + TA +CoTA en différentes proportions sur chacun de ces mélanges. On a ensuite ajouté goutte à goutte la phase aqueuse jusqu'à obtention d'une solution limpide.

Cette quantité ajoutée correspond au pourcentage minimum de la phase hydrophile nécessaire, pour entrer dans la zone micro-émulsionnée. C'est le pourcentage dit d"'entrée".

On continue alors à ajouter la phase hydrophile jusqu'à apparition d'un trouble. La quantité ajoutée correspond au pourcentage dit "de sortie".

Le domaine d'existence de la microémulsion claire et limpide est compris entre deux domaines d'existence d'une émulsion, à l'aspect laiteux.

Ce domaine est représenté par la zone hachurée sur les diagrammes.

Sur les deux diagrammes, il apparaît que la zone d'existence de la microémulsion est relativement large et comprend un pourcentage de phase lipidique de 0 (0 étant exclu) à environ 68% sur la figure 1 et 0 à 72% sur la figure 2.

## Revendications

1. Utilisation d'une microémulsion de type eau-dans-huile destinée à une administration par voie orale pour masquer le goût désagréable d'un principe actif hydrosoluble, pour la fabrication d'un médicament, dans laquelle le principe actif est dissous avant administration dans la phase aqueuse.

2. Utilisation selon la revendication 1, dans laquelle les constituants de la phase lipidique sont hydrodispersibles et liquides à température ambiante.

3. Utilisation selon la revendication 1, **caractérisée en ce que** le(s) constituant(s) de la phase lipidique a (ont) une balance hydrophile-lipophile comprise entre 1,8 et 8,6.

4. Utilisation selon la revendication 1, **caractérisée en ce que** le(s) constituant(s) de la phase lipidique a (ont) une balance hydrophile-lipophile comprise entre 4 et 8,6.

5. Utilisation selon la revendication 4, **caractérisée en ce que** le(s) constituants) de la phase lipidique est(sont) choisis parmi les esters d'acide gras et de polyols, éventuellement polyéthoxylés.

6. Utilisation selon la revendication 5, **caractérisée en ce que** les acides gras sont choisis parmi les acides saturés ou insaturés en C₁₂-C₁₈.

7. Utilisation selon la revendication 5, **caractérisée en ce que** le polyol est choisi parmi le glycérol, les dérivés de polycondensation de celui-ci et le sorbitane.

8. Utilisation selon la revendication 5, **caractérisée en ce que** le degré d'éthoxylation est d'au moins 5 et d'au plus 40 groupes oxyde d'éthylène par molécule de polyol.

9. Utilisation selon la revendication 1, dans laquelle l'agent tensioactif est liquide à température ambiante.

10. Utilisation selon la revendication 1, dans laquelle l'agent tensioactif est un ester d'acide gras de polyol ayant une balance HLB comprise entre 10,5 et 15,6.

11. Utilisation selon la revendication 10, dans laquelle la polyol est choisi parmi le glycérol et les polyglycérols.

12. Utilisation selon la revendication 10, dans laquelle le polyol est le sorbitane,

13. Utilisation selon la revendication 9, dans laquelle l'agent tensioactif est choisi parmi les esters d'acide gras en C₁₂-C₁₈ du sorbitane polyéthoxylé.

14. Utilisation selon la revendication 9, dans laquelle l'agent tensioactif est choisi parmi les esters d'acide gras en C₁₂-C₁₈ du glycérol ou du polyglycérol.

15. Utilisation selon la revendication 1. dans laquelle le cotensioactif est un alcool.

16. Utilisation selon la revendication 15, dans laquelle le cotensioactif est choisi parmi le butanol, le propylène glycol, le glycérol et un éther de polyol, notamment l'éther monoéthylique du diéthylène glycol.

17. Utilisation selon la revendication 9, **caractérisés en ce que** le rapport volumique TA/CoTA est compris entre 1/1 et 50/1.

18. Utilisation selon la revendication 1, **caractérisée en ce que** le principe actif est un sel hydrosoluble de la ranitidine.

19. Procédé de masquage du goût d'un principe actif hydrosoluble à goût désagréable destiné à une administration orale, **caractérisé en ce que** ledit principe actif est dissous avant administration dans la phase aqueuse d'une microémulsion de type eau-dans-huile.

20. Procédé selon la revendication 19, **caractérisé en ce que** les constituants de ladite microémulsion sont tels que définis aux revendications 2 à 18.

21. Composition pharmaceutique comportant une microémulsion de type eau-dans-huile et un sel hydrosoluble de ranitidine, dissous dans la phase aqueuse.

22. Composition salon la revendication 21, **caractérisée en ce que** le sel hydrosoluble de ranifidine est le chlorhydrate de ranitidine.

23. Composition pour masquer le goût désagréable de substances actives hydrosolubles administrées par voie orale, consistant en une microémulsion de type eau-dans-huile comprenant :
- une phase aqueuse contenant un principe actif hydrosoluble représentant de 1 à 30% en volume total de la composition ;
- une phase lipidique constituée d'au moins un ester d'un acide gras en C₁₂-C₁₈ et d'un polyol, ayant une balance hydrophile-lipophile (HLB) comprise entre 4 et 8,6, la phase lipidique représentant de 10 à 72 % du volume total de la composition ;
- au moins un agent tensioactif (TA) ayant une balance hydrophile-lipophile (HLB) comprise entre 10,5 et 15,6 choisi parmi les esters d'acide gras et de polyol, éventuellement éthoxylés ;
- au moins un agent co-tensioactif (CoTA) consistant en un alcool : le rapport TA/CoTA étant compris entre 1 et 50 ;
sous réserve que la phase hydrophile ne soit pas apportée par un organisme vivant auquel la composition est administrée.

24. Composition selon la revendication 23, **caractérisée en ce qu'**elle comprend des constituants tels que définis aux revendications 2 à 18.

## Claims

1. Use of a microemulsion of the water-in-oil type that is intended for oral administration in order to mask the unpleasant taste of a water-soluble active ingredient, in the manufacture of a medicament, in which the active ingredient is dissolved prior to administration in the aqueous phase.

2. Use according to claim 1, in which the constituents of the lipid phase are dispersible in water and liquid at ambient temperature.

3. use according to claim 1, **characterised in that** the constituent(s) of the lipid phase has (have) a hydrophilic-lipophilic balance of from 1.8 to 8.6.

4. Use according to claim 1, **characterised in that** the constituent(s) of the lipid phase has (have) a hydrophilic-lipophilic balance of from 4 to 8.6.

5. Use according to claim 4, **characterised in that** the constituent(s) of the lipid phase is (are) selected from optionally polyethoxylated polyol fatty acid esters.

6. Use according to claim 5, **characterised in that** the fatty acids are selected from saturated or unsaturated C₁₂-C₁₈ acids.

7. Use according to claim 5, **characterised in that** the polyol is selected from glycerol, the polycondensation derivatives thereof and sorbitan.

8. Use according to claim 5, **characterised in that** the degree of ethoxylation is at least 5 and not more than 40 ethylene oxide groups per polyol molecule.

9. Use according to claim 1, in which the surface-active agent is liquid at ambient temperature.

10. Use according to claim 1, in which the surface-active agent is a polyol fatty acid ester having a HLB balance of from 10.5 to 15.6.

11. Use according to claim 10, in which the polyol is selected from glycerol and polyglycerols.

12. Use according to claim 10, in which the polyol is sorbitan.

13. Use according to claim 9, in which the surface-active agent is selected from polyethoxylated C₁₂-C₁₈ sorbitan fatty acid esters.

14. Use according to claim 9, in which the surface-active agent is selected from C₁₂-C₁₈ glycerol or polyglycerol fatty acid esters.

15. Use according to claim 1, in which the cosurfactant is an alcohol.

16. Use according to claim 15, in which the cosurfactant is selected from butanol, propylene glycol, glycerol and a polyol ether, especially diethylene glycol monoethyl ether.

17. Use according to claim 9, **characterised in that** the ratio by volume surface-active agent/cosurfactant is from 1/1 to 50/1.

18. Use according to claim 1, **characterised in that** the active ingredient is a water-soluble ranitidine salt.

19. Method of masking the taste of a water-soluble active ingredient for oral administration having an unpleasant taste, **characterised in that** said active ingredient is dissolved prior to administration in the aqueous phase of a microemulsion of the water-in-oil type.

20. Method according to claim 19, **characterised in that** the constituents of said microemulsion are as defined in claims 2 to 18.

21. Pharmaceutical composition comprising a microemulsion of the water-in-oil type and a water-soluble ranitidine salt dissolved in the aqueous phase.

22. Composition according to claim 21, **characterised in that** the water-soluble ranitidine salt is ranitidine hydrochloride.

23. Composition for masking the unpleasant taste of orally administered water-soluble active substances, consisting of a microemulsion of the water-in-oil type comprising:
- an aqueous phase containing a water-soluble active ingredient, representing from 1 to 30 % of the total volume of the composition;
- a lipid phase constituted by at least one C₁₂-C₁₈ polyol fatty acid ester, having a hydrophilic-lipophilic balance (HLB) of from 4 to 8.6, the lipid phase representing from 10 to 72 % of the total volume of the composition;
- at least one surface-active agent (TA) having a hydrophilic-lipophilic balance (HLB) of from 10.5 to 15.6, selected from optionally ethoxylated polyol fatty acid esters;
- at least one cosurfactant (CoTA) consisting of an alcohol, the TA/CoTA ratio being from 1 to 50; with the proviso that the hydrophilic phase is not supplied by a living organism to which the composition is administered.

24. Composition according to claim 23, **characterised in that** it comprises constituents as defined in claims 2 to 18.

## Patentansprüche

1. Verwendung einer Öl-in-Wasser-Mikroemulsion, die zur oralen Einnahme bestimmt ist, zur Maskierung des unangenehmen Geschmacks eines wasserlöslichen Wirkstoffs, für die Herstellung eines Medikaments, wobei der Wirkstoff vor der Einnahme in der wässrigen Phase gelöst wird.

2. Verwendung nach Anspruch 1, wobei die Bestandteile der Lipidphase bei Raumtemperatur flüssig und in Wasser dispergierbar sind.

3. Verwendung nach Anspruch 1, **dadurch gekennzeichnet, dass** der/die Bestandteil(e) der Lipidphase einen HLB-Wert zwischen 1,8 und 8,6 aufweist/aufweisen.

4. Verwendung nach Anspruch 1, **dadurch gekennzeichnet, dass** der/die Bestandteil(e) der Lipidphase einen HLB-Wert zwischen 4 und 8,6 aufweist/aufweisen.

5. Verwendung nach Anspruch 4, **dadurch gekennzeichnet, dass** der/die Bestandteil(e) der Lipidphase aus möglicherweise polyethoxylierten Estern aus Fettsäuren und Polyolen gewählt wird/werden.

6. Verwendung nach Anspruch 5, **dadurch gekennzeichnet, dass** die Fettsäuren aus gesättigten oder ungesättigten C₁₂- bis C₁₈-Säuren gewählt werden.

7. Verwendung nach Anspruch 5, **dadurch gekennzeichnet, dass** das Polyol aus Glycerin, dessen Polykondensationsderivaten sowie Sorbitan gewählt wird.

8. Verwendung nach Anspruch 5, **dadurch gekennzeichnet, dass** das Polyol einen Ethoxylierungsgrad von mindestens 5 und höchstens 40 Ethylenoxidgruppen pro Molekül aufweist.

9. Verwendung nach Anspruch 1, wobei das Tensid bei Raumtemperatur flüssig ist.

10. Verwendung nach Anspruch 1, wobei es sich bei dem Tensid um einen Ester aus einer Fettsäure und einem Polyol handelt, welcher einen HLB-Wert zwischen 10,5 und 15,6 aufweist.

11. Verwendung nach Anspruch 10, wobei das Polyol aus Glycerin und Polyglycerinen gewählt wird.

12. Verwendung nach Anspruch 10, wobei es sich bei dem Polyol um Sorbitan handelt.

13. Verwendung nach Anspruch 9, wobei das Tensid aus den C₁₂- bis C₁₈-Fettsäureestern des polyethoxylierten Sorbitans gewählt wird.

14. Verwendung nach Anspruch 9, wobei das Tensid aus den C₁₂- bis C₁₈-Fettsäureestern des Glycerins oder des Polyglycerins gewählt wird.

15. Verwendung nach Anspruch 1, wobei es sich bei dem Cotensid um einen Alkohol handelt.

16. Verwendung nach Anspruch 15, wobei das Cotensid aus Butanol, Propylenglykol, Glycerin, sowie einem Polyolether, insbesondere Diethylenglykolmonoethylether, gewählt wird.

17. Verwendung nach Anspruch 9, **dadurch gekennzeichnet, dass** das Volumenverhältnis TA/CoTA zwischen 1/1 und 50/1 beträgt.

18. Verwendung nach Anspruch 1, **dadurch gekennzeichnet, dass** es sich bei dem Wirkstoff um ein wasserlösliches Salz des Ranitidins handelt.

19. Verfahren zur Maskierung des Geschmacks eines unangenehm schmeckenden Wirkstoffs, welcher zur oralen Einnahme bestimmt ist, **dadurch gekennzeichnet, dass** der Wirkstoff vor der Einnahme in der wässrigen Phase einer Öl-in-Wasser-Mikroemulsion gelöst wird.

20. Verfahren nach Anspruch 19, **dadurch gekennzeichnet, dass** die Bestandteile der Mikroemulsion den Definitionen der Ansprüche 2 bis 18 entsprechen.

21. Pharmazeutische Zusammensetzung, die eine Öl-in-Wasser-Mikroemulsion sowie ein wasserlösliches Ranitidinsalz, welches in der wässrigen Phase gelöst ist, umfasst.

22. Zusammensetzung nach Anspruch 21, **dadurch gekennzeichnet, dass** es sich bei dem wasserlöslichen Ranitidinsalz um Ranitidinchlorhydrat handelt.

23. Zusammensetzung zur Maskierung des unangenehmen Geschmacks wasserlöslicher Wirkstoffe zur oralen Einnahme, bestehend aus einer Mikroemulsion, die folgende Elemente umfasst:
- eine wässrige Phase, die einen wasserlöslichen Wirkstoff enthält und 1 bis 30 % des Gesamtvolumens der Zusammensetzung ausmacht;
- eine Lipidphase, die aus mindestens einem Ester aus einer C₁₂- bis C₁₈-Fettsäure und einem Polyol besteht und einen HLB-Wert zwischen 4 und 8,6 aufweist, wobei die Lipidphase 10 bis 72 % des Gesamtvolumens der Zusammensetzung ausmacht;
- mindestens ein Tensid (TA), welches einen HLB-Wert zwischen 10,5 und 15,6 aufweist und aus den möglicherweise ethoxylierten Estern aus Fettsäuren und Polyol gewählt wird;
- mindestens ein Cotensid (CoTA), wobei es sich um einen Alkohol handelt und das Verhältnis TA/CoTA zwischen 1 und 50 beträgt;
unter dem Vorbehalt, dass die wässrige Phase nicht von dem Lebewesen geliefert wird, welchem die Zusammensetzung verabreicht wird.

24. Zusammensetzung nach Anspruch 23, **dadurch gekennzeichnet, dass** sie Bestandteile umfasst, die den Definitionen der Ansprüche 2 bis 18 entsprechen.
